# EUROPEAN PATENT APPLICATION

(11) **EP 4 763 075 A1**
(43) Date of publication of application: **24.06.2026**
(21) Application number: 24221647.1
(22) Date of filing: 19.12.2024
(51) Int. Cl.: A61B 5/103, A61B 5/00

(54) **MEDICAL DEVICE CONTROL USING A PRESSURE SENSOR ARRAY**

(71) Applicant: Koninklijke Philips N.V., 5656 AE Eindhoven (NL)
(72) Inventor: VAN EE, Raymond, Eindhoven (NL); MASON, Jonathan David, Eindhoven (NL); DENISSEN, Adrianus Johannes Maria, Eindhoven (NL); SENEGAS, Julien Thomas, Eindhoven (NL); KRUEGER, Sascha, Eindhoven (NL); WIEMKER, Rafael, Eindhoven (NL); WIRTZ, Daniel, Eindhoven (NL)
(74) Representative: Philips Intellectual Property & Standards

(57) **Abstract**

The present disclosure relates to a medical imaging device (1). The medical imaging device having an array of pressure sensors (3) arranged on top of the patient support surface (2). The output of the array of pressure sensors is provided to a control unit (5). The control unit derives control commands from the output of the array of pressure by comparing the received outputs to a set of predefined outputs linked to commands. The operation of the medical imaging device is based on the commands derived by the control unit.

## Description

### FIELD OF THE INVENTION

The present disclosure relates to the control of a medical device, in particularly by using a pressure sensor array.

### BACKGROUND OF THE INVENTION

Medical imaging devices, especially those of the magnetic resonance (MR) and computed tomography (CT) are typically complex requiring a skilled operator to perform challenging tasks. Other imaging techniques, such as X-ray could for some exams require a specific and more complex operating tasks as well.

One of the challenges encountered by operators is that their control interface might be in a separate location or might not be reachable to input commands while they are working on the correct positioning of the patient for the exam at hand. This may delay the set-up process, which should be as short as possible in view of hospital economics requiring as many exams as possible in a given period of time. Also, patients may experience more anxiety if they need to wait longer than strictly necessary before the actual imaging starts. Further, the time gap between completion of the patient preparation and the start of the actual imaging should be as short as possible to reduce the risk of undesired patient movement. Undesired patient movement may lead to clinically less relevant images and re-takes.

Pressure sensor arrays placed on the patient couch of a medical imaging device are known as such, e.g. from US 2023/0221194 A1 which discloses a two-dimensional optical waveguide pressure sensor array comprising two or more row optical waveguides; two or more column optical waveguides, wherein the row optical waveguides and the column optical waveguides are deformable and arranged in a planar array to define a sensor in the crosspoints. This sensor array is disclosed to be usable for measuring and monitoring the specific location and movement of persons, e.g. persons during MRI scanning.

### SUMMARY OF THE INVENTION

To better address one or more of these concerns, there is provided, in a first aspect, a medical imaging device comprising a patient support surface and an array of pressure sensors arranged on top of the patient support surface. When in use, the array of pressure sensors is arranged between a patient and the patient support surface, the array of pressure sensors having an output indicative of the pressure sensed at each sensor. Further, the medical imaging device comprises a control unit. This control unit is arranged, in an execution mode, to receive the output from the array of pressure sensors, derive a control command from the received output by comparing the received outputs to a set of predefined outputs linked to commands, and control the operation of the medical imaging device based on the received output from the array of pressure sensors.

In the medical imaging device according to the invention the patient support acts as a user input device. By engaging with (e.g. tapping on) the patient support surface, control commands can be provided to the medical imaging device. This user input device is in the direct vicinity of the patient and thus of the operator during patient set-up. The operator may therefore provide control commands to the medical imaging device while being in the direct vicinity of the patient.

In an embodiment, deriving a control command is dependent on a temporal phase of the imaging procedure. In other words, the interpretation of the sensor outputs by the control unit might be different based on the phase of the examination. E.g. a double tap received before the imaging starts may be interpreted as being part of inputting parameters relevant to the imaging settings by the operator, while receiving such a double tap during the examination may be interpreted as inputting parameters by the patient relevant for his or her well-being during the examination. Interpreting the sensor output based on the temporal phase of the imaging procedure reduces the chances that an input is misinterpreted. At the same time, the number of control commands derivable from the output of the array of pressure sensors might be increased without increasing the complexity of the engagement patters of an operator or patient with the sensor array as e.g. double tapping on a specific location may have a different interpretation, thus being derived to correspond to a different control command in dependence on the temporal phase of the examination. There might be many temporal phases as part of the total imaging procedure. In a practical embodiment, the number of temporal phases might be 2, 3, or 4. In an embodiment in which the number of temporal phases is 4, there might be an examination set-up phase in which the operator provides commands to the imaging device relevant for the specific examination and/or the specific patient, an intermediate phase in which the operator is on his or her way to a control room, an examination execution phase which commences once the operator is in the control and has confirmed that the execution of the exam might start, and a post-examination phase following the actual imaging.

In a preferred embodiment, the temporal phase of the imaging procedure is one of examination set-up and examination execution. By splitting the imaging procedure into these two phases an easy to understand phasing for both the operator and patient is achieved which connects to on the one hand the phase in which the operator will provide commands to the medical imaging device and on the other hand the phase in which the patient will provide commands to the medical imaging device.

In an embodiment, the medical imaging device comprises a camera sensor for observing the patient support surface, wherein the control unit is further arranged to receive the output of the camera sensor and to determine, based on the camera output, whether the pressure variations originate from a patient positioned on the patient support or from an operator standing close to the patient support. This camera sensor may be a workflow support camera attached to the ceiling above the patient support a workflow support camera located on the medical imaging device, e.g. on the gantry of a CT or MR device. Workflow support cameras are well-known as such. The inventors realized that these cameras may be used to support the interpretation of inputs provided via the pressure sensor array as contemplated in this invention. Alternatively, a dedicated camera sensor might be used which is not providing information to a workflow support system. The output obtained from the camera sensor might be interpreted to establish the phase of the imaging. E.g. when based on the camera system output it is detected that an operator is standing next to the patient support it might be concluded that the patient set-up phase is still ongoing. In another example, the output of the camera sensor might indicate that the operator has left the examination room a phase following the examination set-up phase has commenced.

In an embodiment, the medical imaging device does not only comprise an execution mode as disclosed above, but in addition comprises a training mode as well. In this training mode inputs might be provided to the pressure sensor array which are linked, e.g. by the operator or an installation engineer, to specific control commands. This allows the behavior of the medical imaging device, or in other words the way it reacts to certain inputs provided via the pressure sensor array to be adapted to site or installation specific requirements. In the training phase the controller of the medical imaging device may, so to speak, learn or relate some specific output from the array of pressure sensors to a specific control command.

In an embodiment, deriving a control command from the output from the pressure sensor array as received by the control unit comprises recognizing multiple, time separated pressure variations. In an example this might correspond to double tapping at a singe location.

In an embodiment, deriving a control command from the output from the pressure sensor array as received by the control unit comprises recognizing multiple, spatially separated pressure variations. In an example this might correspond to taps at different locations on the patient support surface within a specific time period.

In an embodiment the localization of a field of view of a localizer scan of the medical imaging device may be indicated by tapping the patient support surface by the operator during the examination set-up phase. This may replace using a laser pointer as known in current medical imaging systems. The laser pointer is less patient friendly than tapping on the patient support. The operator might tap on the patient support surface to indicate the field of view of the localizer scan.

In an embodiment the medical imaging device is an MR imaging device and wherein the control command is the indication of the definition of slices and stacks. As is known in the field, a slice is a cross-section and a stack is a set of slices. More precisely, a slice is a plane through a patient with a specific thickness. This thickness corresponds to the depth of the section of the patient being imaged. In this embodiment the control unit derives the definition of slices and stacks from the output of the array of pressure sensors.

In an embodiment which is specifically relevant for exams requiring contrast agent to be supplied to the patient, the control command is the indication of a bolus direction in a contrast agent scan. E.g. in the case a stack of images is to be obtained, the stack of images should be taken in the direction of the flow of the contrast agent. The operator might indicate the direction of the bolus direction using a swipe over the patient support surface.

It has been realized by the inventors that not only an operator might benefit from using the patient support surface as a user interface. There might be controls which might be inputted by both the operator or the patient, or the patient only. In an embodiment, the control command derived from the pressure sensor array output is the adjustment of a light setting. It is known as such that some people feel more at ease in a dimmed light environment, while others prefer a brightly lit environment. It might be beneficial for the comfort of the patient that he or she may control the light settings in the examination room once the examination parameters are set by the operator.

In an embodiment, the control command derived from the pressure sensor array output is the adjustment of an entertainment setting such as a change of volume or a change of channel. Especially in examination taking a significant amount of time, such as MR imaging, the patient might be provided with entertainment, e.g. to provide comfort to the patient, to shorten the experienced duration of the examination and / or to distract the patient from situations the patient might be anxious of such as being placed in the bore of an MR imaging device. When a patient is provided with entertainment during the examination, it is particularly helpful to allow the patient to control this entertainment by e.g. switching audio or video channels and/or adjusting sound levels. The option to adjust sound levels is particularly relevant during examinations in which significant noise is generated by the imaging device. By having the patient support available as input device to the patient, no separate input device is needed.

In an embodiment, the control command derived from the pressure sensor array output is the adjustment of the patient table position. The adjustment of the table position is a common activity for the operator during the examination set-up phase, e.g. to lower the table to allow the patient to get onto the table without difficulties and to return to the table to the height required for the examination once the patient in on the table. The top side of the patient table is the patient support surface. Integrating the control of the patient table in the top side of the patient table simplifies the work of the operator and removes the need for a second user interface or control unit. In another example the table position adjustment might be into or out of an examination area of the medical imaging device

In an embodiment, the array of pressure sensors is removable from the patient table. This allows for easy cleaning of the pressure sensor array and replacement of the pressure sensor array in case of malfunctioning. Further, different types of pressure sensor arrays might be provided for use with different types of examination. By having a removable pressure sensor array flexibility is added to the medical device installation.

In an alternative embodiment, the array of pressure sensors is integral with the patient table. Such an integrated solution allows for a well-defined relationship between the individual pressure sensors and the coordinate system of the medical imaging device. This may make the interpretation of the received output of the pressure sensor array by the controller more straightforward or at least less complicated as specific sensor nodes in the sensor array are linked to a known location on the patient support surface.

### BRIEF DESCRIPTION OF THE DRAWINGS

A detailed description will now be given, by way of example only, with reference to the accompanying drawings, in which:-
Fig. 1 shows a schematic side view of a medical imaging device; and
Fig. 2 shows a schematic top view of a patient support table comprising a pressure sensor array.

### DETAILED DESCRIPTION OF EMBODIMENTS

Like numbered elements in these figures are either equivalent elements or perform the same function. Elements which have been discussed previously will not necessarily be discussed in later figures if the function is equivalent.

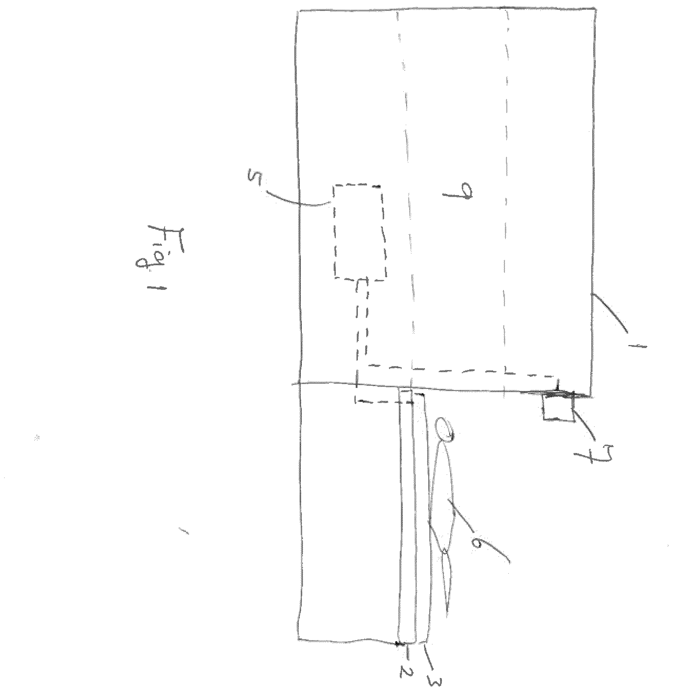

Fig. 1 shows a medical imaging device 1. In this example the medical imaging device is a magnetic resonance imaging (MRI) device having a so-called bore in which at least the part of a patient which is to be imaged needs to reside during imaging. MRI devices are well-known to the person skilled in the art. In this bore the imaging area 9 is located. Although the invention is illustrated in this example as a medical imaging device being an MRI, the invention might be implemented in other medical imaging devices as well, e.g. Computed Tomography (CT) and X-ray devices having a patient support surface.

The medical imaging device 1 comprises a patient support surface 2. In some embodiments this patient support surface might be moveable, e.g. the patient support surface might be brought in a position in which a patient can get on the surface easily and be returned to a height at which the imaging is to take place and/or at which the operator 8 may work to prepare the patient for examination in an ergonomic position. Further, the patient support surface might be brought to a height at which an operator can work ergonomically. In addition to the height adjustment, the patient support surface might be moveable into and out of an imaging area 9.

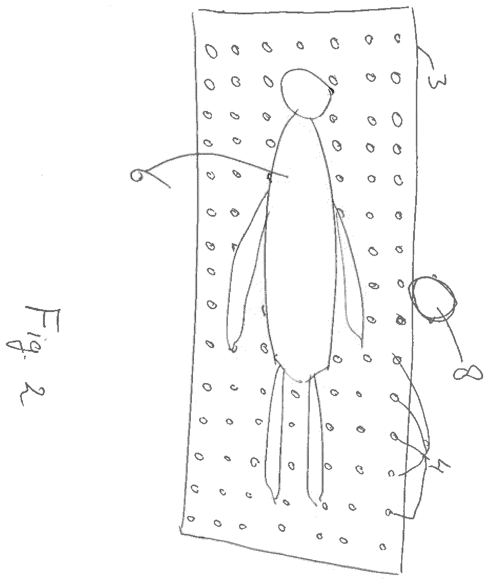

A pressure sensor array 3 is placed on top of patient support surface 2. As illustrated in Fig. 2, the pressure sensor array comprises individual sensors 4. During use of the medical imaging the pressure sensor array is placed between patient 6 and the patient support surface. As is visible in Fig. 2, not all pressure sensors will be covered by the patient. These pressure sensors not covered by the patient are available to either the operator 8 or the patient to manipulate the sensors, that is to touch the sensors and apply force to them, such that a pressure sensor output changes. This pressure sensor output might be interpreted by control unit 5 as a control command.

The medical device 1 may further comprise a camera sensor 7 which is arranged to observe the patient table during examination set-up and/or examination execution. In some embodiments there might be more than one camera sensor. More than one camera sensor might be applied as a single camera sensor is not able to observe both the examination set-up area and the imaging area. Alternatively, multiple camera sensors might be employed to reduce the risk of the field of view of the camera sensor being blocked such that the camera sensor might not provide usable outputs. In some imaging set-ups, complex workflow and patient observation systems are used employing multiple camera sensors to obtain 3D images of the patient on the patient support surface. In the context of the present invention, the type and/or number of cameras is not relevant, as long as their output might be used for the purpose required by the invention.

The control unit 5 is to receive the output of the array of pressure sensors 3, that is a signal indicative of the pressure acting upon each of the pressure sensors 4. In some embodiment the control unit will receive the output of camera sensor 7 as well.

The control unit may derive a position of the patient and other information regarding the patient, such as his or her respiration and cardiac cycles from the output of the array of pressure sensors, as is known in the field, e.g. from US 2021/0121139 A1.

According to the present invention the control unit may derive control commands from the received output of the array of pressure sensors. In particular, the control unit may recognize specific patterns in the signal indicative of a pressure acting upon one or more pressure sensors in the pressure sensor array. The control unit may correlate the output of the camera sensor with the output of the pressure sensor array, e.g. to select pressure sensors in reach of either the patient or the operator, or to select pressure sensors touched by the patient or operator, for analysis to derive control commands from the output of the array of pressure sensors.

It is understood that that the derivation of control commands may be dependent on who is providing the pressure to the pressure sensor, e.g. the operator 8 or the patient 2, as well as the moment in the examination procedure. For example, during examination set-up the control commands might be different from the ones during examination execution. In some embodiments only the input of, that is the pressure variations induced by, the operator might be taken into account during the examination set-up phase. The camera sensor output might be used to relate a specific sensor output to either the patient or the operator. Alternatively, or in addition, the patient position as derived from the pressure sensor array output might be used to discriminate between the operator providing pressure variations to one or more pressure sensors in the pressures sensor array and the patient providing such pressure variations. Based on the derived position of the patient on the patient support, the controller might derive which sensors are in in location within reach of the patient's hand or foot. Similarly, the camera sensor output might be used by the controller to derive which sensors are in a location within reach of the operator.

The output from the array of pressure sensors might be interpreted to derive control commands by de controller based on a temporal phase of the imaging procedure. Typically, the total workflow in a medical imaging examination comprises multiple phases, such as, but not limited to, an imaging set-up phase and an examination execution phase. In the imaging set-up phase the operator might position the patient into the pose required for the examination at hand. As part of this imaging set-up phase the operator might need to indicate to the imaging device the which parts of the patient need to be imaged. An example of this indication activity might be the definition of slices and stacks in an MRI procedure. Another example might be the definition of the direction of the bolus in a contrast agent scan. In order to optimize imaging in an examination involving contrast agent being administered to a patient and multiple, in examinations wherein spatially separated images of a patient are to be acquired, the start and end locations of the imaging area are to be defined such that the imaging starts at the location at which the contrast agent arrives and "flows" with the contrast agent to end of the end location of the imaging. A further example might be the indication of relevant keypoints, e.g. the umbilicus of a patient.

The controller may distinguish between temporal phases of the examining procedure based on the information from the imaging device. An imaging set-up phase might be the period up to the activation of the imaging device to image the patient. The examination execution phase might be the period during which the imaging device is actually imaging the patient. The duration of the examination execution phase will be dependent on the type of medical imaging device and the specific examination at hand. Alternatively, or in addition to the fact if the imaging device is performing the actual imaging of the patient, the controller might use the output of the camera sensor to establish the temporal phase of the imaging procedure. E.g. the examination set-up phase might be considered to be ongoing while the operator is standing near or next to the patient support.

During the examination execution phase the operator is typically not in the direct vicinity of the of the medical imaging device. Any derived control commands are therefore necessary inputted by the patient. During the imaging execution the patient might provide emergency signals to the medical imaging device. From such signals the medical imaging device controller might derive a procedure stop command. Especially in examination procedures taking a longer time to complete, e.g. certain MRI examinations, the patient might be provided with entertainment such as audio or video. The patient might control this entertainment during the examination execution phase by moving or tapping with a limb which is not being imaged. It is further known that light settings might be varied during an examination execution. By allowing the patient to provide light control commands via the patient support surface the patient might select the setting at which he or she feels most at ease. For most, if not all, exams it is desired that the patient feels at ease during the examination execution as this will limit or at least reduce the risk of undesired patient movement.

In a practical embodiment, the format of the input to the pressure sensor array arranged on the patient support might be comparable to the operation of smart devices, e.g. via tapping, double tapping, single finger swiping and / or double finger swiping. In addition, or in the alternative, the input to the pressure sensor array might be provided by using two hands or two feet simultaneously. A double tapping input is an example of a time separated pressure variation inputted to the pressure sensor array. A swipe input involves the actuation of multiple sensors in the pressure sensor array and is an example of a spatially separated pressure variation.

Various functions described herein can be implemented in hardware, software, or any combination thereof. If implemented in software, the functions can be stored on or transmitted over as one or more instructions or code on a computer-readable medium. Computer-readable media include computer-readable storage media. Computer-readable storage media can be any available storage media that can be accessed by a computer. By way of example, and not limitation, such computer-readable storage media can comprise FLASH storage media, RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, include compact disc (CD), laser disc, optical disc, digital versatile disc (DVD), floppy disk, and Blu-ray disc (BD), where disks usually reproduce data magnetically and discs usually reproduce data optically with lasers. Further, a propagated signal may be included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio and microwave are included in the definition of communication medium. Combinations of the above should also be included within the scope of computer-readable media.

Alternatively, or in addition, the functionally described herein can be performed, at least in part, by one or more hardware logic components. For example, and without limitation, illustrative types of hardware logic components that can be used include Field-programmable Gate Arrays (FPGAs), Program-specific Integrated Circuits (ASICs), Program-specific Standard Products (ASSPs), System-on-a-chip systems (SOCs), Complex Programmable Logic Devices (CPLDs), etc.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The mere fact that certain measures are recited in mutually different dependent claims does not indicate that a combination of these measures cannot be used advantageously.

Any reference signs in the claims should not be construed as limiting the scope.

### REFERENCE SIGNS LIST

- 1: medical imaging device
- 2: patient support image
- 3: pressure sensor array
- 4: pressure sensor
- 5: control unit
- 6: patient
- 7: camera sensor
- 8: operator
- 9: imaging area

## Claims

1. A medical imaging device (1) comprising
a patient support surface (2) and
an array of pressure sensors (3) arranged on top of the patient support surface such that, when in use, the array of pressure sensors is arranged between a patient and the patient support surface, the array of pressure sensors having an output indicative of the pressure sensed at each sensor (4), and
a control unit (5)
wherein the control unit is arranged, in an execution mode, to
receive the output from the array of pressure sensors,
derive a control command from the received output by comparing the received outputs to a set of predefined outputs linked to commands, and
control the operation of the medical imaging device based on the received output from the array of pressure sensors.

2. The medical imaging device according to claim 1, wherein the step of deriving a control command is dependent on a temporal phase of the imaging procedure.

3. The medical imaging device according to claim 2, wherein the phase of the imaging procedure is one of
- examination set-up;
- examination execution.

4. The medical imaging device according to any of the preceding claims, wherein the imaging device further comprises a camera sensor (7) for observing the patient support surface, wherein the control unit is further arranged to receive the output of the camera sensor and to determine, based on the camera output, whether the pressure variations originate from a patient (6) positioned on the patient support or from an operator (8) standing close to the patient support.

5. The medical imaging device according to any of the preceding claims, wherein the control unit, in a training mode, is arranged to relate received output from the array of pressure sensors to a control command.

6. The medical imaging device according to any of the preceding claims, wherein deriving a control command comprises recognizing multiple, time separated pressure variations.

7. The medical imaging device according to any of the preceding claims, wherein deriving a control command comprises recognizing multiple, spatially separated pressure variations.

8. The medical imaging device according to any of the preceding claims, wherein the control command is the localization of a field of view of a localizer scan of the medical imaging device.

9. The medical imaging device according to any of claims 1 to 7 wherein the imaging device is an MR imaging device and wherein the control command is the indication of the definition of slices and stacks.

10. The medical imaging device according to any of claims 1 to 7 wherein the control command is the indication of a bolus direction in a contrast agent scan.

11. The medical imaging device according to any of the preceding claims, wherein the control command is the adjustment of a light setting.

12. The medical imaging device according to any of the preceding claims, wherein the control command is the adjustment of an entertainment setting such as a change of volume or a change of channel.

13. The medical imaging device according to any of the preceding claims, wherein the control command is the adjustment of the patient table position.

14. The medical imaging device according to any of the preceding claims, wherein the array of pressure sensors is removable from the patient table.

15. The medical imaging device according to any of the preceding claims, wherein the array of pressure sensors is integral with the patient table.
